(19) Europäisches Patentamt
European Patent Office
Office européen des brevets

(11) Veröffentlichungsnummer: **0 097 364**
**B1**

# (12) EUROPÄISCHE PATENTSCHRIFT

(45) Veröffentlichungstag der Patentschrift: **28.01.87**

(51) Int. Cl.⁴: **C 12 N 11/02**, C 12 N 11/00, G 01 N 33/53, C 07 F 7/02, C 07 F 7/18, C 08 G 77/38

(21) Anmeldenummer: **83106047.0**

(22) Anmeldetag: **21.06.83**

## (54) Stabilisierte biochemische Suspensionspräparate.

(30) Priorität: **21.06.82 DE 3223101**

(43) Veröffentlichungstag der Anmeldung: **04.01.84 Patentblatt 84/1**

(45) Bekanntmachung des Hinweises auf die Patenterteilung: **28.01.87 Patentblatt 87/5**

(84) Benannte Vertragsstaaten: **CH DE FR GB IT LI SE**

(56) Entgegenhaltungen:
**DE - A - 1 915 970**
**DE - A - 1 944 418**
**DE - A - 2 106 215**
**DE - A - 2 905 657**
**DE - B - 2 758 507**
**US - A - 3 652 761**

(73) Patentinhaber: **Fraunhofer-Gesellschaft zur Förderung der angewandten Forschung e.V., Leonrodstrasse 54, D-8000 München 19 (DE)**
Patentinhaber: **Von Stetten, Otto, Blaubeurer Strasse 91, D-7933 Schelklingen (DE)**

(72) Erfinder: **von Stetten, Otto, Dr., Blaubeurer Strasse 91, D-7933 Schelklingen (DE)**
Erfinder: **Patzelt, Helmut, Dr., Obere Dorfstrasse 33, D-8702 Kist (DE)**
Erfinder: **Schmidt, Helmut, Dr., Tilman-Riemenschneider-Strasse 15, D-8706 Höchberg (DE)**

(74) Vertreter: **Barz, Peter, Dr. et al, Patentanwälte Dr. V. Schmied-Kowarzik Dipl.-Ing. G. Dannenberg Dr. P. Weinhold Dr. D. Gudel Dipl.-Ing. S. Schubert Dr. P. Barz Siegfriedstrasse 8, D-8000 München 40 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft stabilisierte biochemische Suspensionspräparate, die sich insbesondere für immunchemische Verfahren, z.B. Radio-, Enzym- oder Fluoreszenzimmunoassays, aber auch als Bioreaktoren eignen.

Der Einsatz von an Festkörpern fixierten biochemischen Materialien, z.B. Antigenen, Antikörpern, Hormonen, Aminosäuren, Haptenen, Proteinen oder Enzymen, in immunchemischen Verfahren ist bekannt. Für Immunosassays hat sich hierbei insbesondere die Verwendung von Teströhrchen bewährt, deren Innenoberfläche mit dem biochemischen Material beschichtet ist (Coated Tubes); siehe z.B. Catt et al, Science 158 (1967) S. 1570/1571.

Bei dem in der US-A-3 652 761 beschriebenen Präparat sind biochemische Materialien über einen (monomeren) Silankuppler an die Oberfläche von anorganischen Trägerstoffen gebunden. Diese Präparate weisen somit kupplungsfähige funktionelle Gruppen nur in einer dünnen oberflächlichen Monoschicht auf, die entsprechend abriebempfindlich ist. Auch in der DE-A-2 106 215 werden (monomere) Silane als Haftvermittler zur Verbesserung der Haftung zwischen einem siliciumhaltigen Trägermaterial und einem Carboxylgruppen enthaltenden Polymerisat verwendet, an das seinerseits ein Enzym kovalent gebunden ist. Hier erfolgt somit keine Kupplungsreaktion zwischen dem biochemischen Material (Enzym) und den funktionellen Gruppen des Silans.

Aus der DE-A-2 758 507 sind Coated-Tube-Systeme bekannt, die Kieselsäureheteropolykondensate als Beschichtung aufweisen. Diese Coated-Tube-Systeme haben jedoch den Nachteil, dass ihre Bindungskapazität zum Ankoppeln der biochemischen Materialien beschränkt ist bzw. entsprechend grosse Oberflächen notwendig sind. Ferner ist die Kinetik der Coated-Tube-System diffusionsbestimmt, d.h. die Messungen werden von der Diffusionsgeschwindigkeit des biochemischen Substrats zur beschichteten Röhrchenoberfläche beeinflusst und dadurch zeitaufwendig und ungenau.

Ziel der Erfindung ist es daher, stabilisierte biochemische Materialien bereitzustellen, die beim Einsatz in biologischen oder biochemischen Verfahren eine beliebig hoch dosierbare kopplungsfähige Oberfläche zur Verfügung stellen können und eine Kinetik entsprechend einem homogenen System, d.h. ohne Diffusionsprobleme, ermöglichen.

Diese Aufgabe wird mit einem stabilisierten biochemischen Suspensionspräparat gemäss Hauptanspruch gelöst.

Die erfindungsgemässen Suspensionspräparate haben den Vorteil, dass sie die Eigenschaften von an Festkörpern fixierten biochemischen Materialien, d.h. insbesondere die Stabilisierung der biochemischen Substanzen, und die Eigenschaften einer Flüssigkeit, z.B. genaue und einfache Dosierbarkeit, in sich vereinigen. Die Suspensionen sind über längere Zeit stabil und können als Quasi-Flüssigkeiten leicht mit einer Pipette dosiert werden. Gegebenenfalls wird vor dem Gebrauch aufgeschüttelt. Nach Durchführung der Tests oder Ansätze lassen sich die Suspensionsteilchen andererseits wieder leicht abtrennen, z.B. durch Zentrifugieren oder Filtrieren.

Die erfindungsgemässen Suspensionspräparate haben eine ausserordentlich hohe und beliebig steuerbare spezifische Oberfläche, die für die Ankopplung der biochemischen Materialien zur Verfügung steht. Aufgrund ihrer Eigenschaft als Quasi-Flüssigkeiten spielen auch Diffusionsvorgänge im Gegensatz zu herkömmlichen Coated-Tube-Systemen keine Rolle, sondern es liegt eine homogene Kinetik vor, die eine schnelle und genaue Messung ermöglicht.

Die erfindungsgemässen Suspensionspräparate enthalten Teilchen eines funktionelle Gruppen aufweisenden Kieselsäureheteropolykondensats, an dessen funktionelle Gruppen biochemische Materialien kovalent gebunden sind, in einem wässrigen Medium suspendiert. Gewöhnlich beträgt der Gehalt an Kieselsäureheteropolykondensat-Teilchen 0,1 bis 50, vorzugsweise 0,5 bis 30 Gewichtsprozent, bezogen auf das Gewicht der Suspension, jedoch sind für spezielle Anwendungen auch Konzentrationen ausserhalb dieser Bereiche anwendbar.

Die durchschnittliche Grösse der Kieselsäureheteropolykondensat-Teilchen beträgt gewöhnlich 0,1 bis 10 µm, vorzugsweise 0,1 bis 5 µm und insbesondere 0,1 bis 1 µm, jedoch sind für spezielle Anwendungen auch Teilchengrössen ausserhalb dieser Bereiche anwendbar.

Als wässriges Medium können Wasser oder wässrige Lösungen, z.B. Pufferlösungen, verwendet werden. Gegebenenfalls und je nach dem Verwendungszweck enthalten die Suspensionspräparate noch weitere Zusätze, z.B. Suspensionshilfsmittel, wie Polyalkylenglykole, Sucrose oder Triethanolamin; Puffer, wie Phosphat-, Barbital- oder Trispuffer; Konservierungsmittel, wie Natriumazid, Merthiolat, Benzoesäurederivate oder quaternäre Ammoniumverbindungen; oder sonstige übliche Additive, z.B. Zusätze, die die biochemische Aktivität stabilisieren, wie Polyvinylalkohol oder Proteine.

Die Kieselsäureheteropolykonsensat-Teilchen werden hergestellt durch hydrolytische Polykonsensation von einem oder mehreren Silanen oder Polysiloxanen, wobei mindestens ein Silan oder Polysiloxan eine mit biochemischen Materialien reaktive funktionelle Gruppe aufweist, in einem organischen Lösungsmittel gegebenenfalls in Gegenwart eines Kondensationskatalysators, Zerkleinern des erhaltenen Kieselsäureheteropolykondensats und Ankoppeln des biochemischen Materials nach üblichen Methoden der organischen Chemie.

Die Kieselsäureheteropolykondensate werden erhalten durch hydrolytische Polykondensation von

a) mindestens einem vollständig hydrolysierbaren Silan der allgemeinen Formel I

$SiX_4$ (I)

in der X Wasserstoff, Halogen, Hydroxy, Alkoxy, Acyloxy oder $-NR_2$ (R = Wasserstoff und/oder Alkyl) bedeutet, jedoch nicht alle Reste X Wasserstoff sind, und

b) mindestens einem organofunktionellen Silan der allgemeinen Formel II

$R'_a(R''Y)_bSiX_{(4-a-b)}$ (II)

in der X die vorstehende Bedeutung hat, R′ Alkyl, Alkenyl, Aryl, Arylalkyl, Alkylaryl, Arylalkenyl oder Alkenylaryl bedeutet, R″ Alkylen, Phenylen, Alkylenphenylen oder Alkenylen darstellt, wobei diese Reste durch Sauerstoff- oder Schwefelatome oder -NH-Gruppen unterbrochen sein können, Y Halogen oder eine gegebenenfalls substituierte Amino-, gegebenenfalls substituierte Anilino-, Amid-, Polyamid-, Aldehyd-, Keto-, Carboxy-, Hydroxy-, Polyol-, Mercapto-, Cyano-, Diazo-, Carbonsäurealkylester-, Sulfonsäure-, Phosphorsäure-, Acryloxy-, Methacryloxy-, Epoxid-, Vinyl-, N-Maleinimido- oder N-(5-Iminofuran-2-on)-Gruppe ist, a den Wert 0, 1 oder 2 und b den Wert 1, 2 oder 3 haben, wobei (a + b) den Wert 1, 2 oder 3 hat, sowie gegebenenfalls den folgenden Komponenten c) und/oder d):

c) mindestens einem Organosilan der allgemeinen Formel III

$R'_bSiX_{(4-b)}$ (III)

in der X, R′ und b die vorstehende Bedeutung haben;

d) mindestens einem im Reaktionsmedium löslichen schwer-flüchtigen Oxid oder mindestens einer im Reaktionsmedium löslichen, ein schwerflüchtiges Oxid bildenden Verbindung eines Elements der Hauptgruppen Ia bis Va oder der Nebengruppen IVb oder Vb des Periodensystems.

Das Kieselsäureheteropolykondensat enthält hierbei gewöhnlich 40 bis 95 Gewichtsprozent der Komponente a), 5 bis 50 Gewichtsprozent der Komponente b), 0 bis 40 Gewichtsprozent der Komponente c) und 0 bis 30 Gewichtsprozent der Komponente d), jeweils bezogen auf Siloxan- oder Oxideinheiten im fertigen Kondensat. Die Komponente a) wird vorzugsweise in einer Menge von mindestens 50, insbesondere 70 Gewichtsprozent und höchstens 90, insbesondere 80 Gewichtsprozent verwendet. Die Komponente b) wird vorzugsweise in einer Menge von mindestens 10, insbesondere 20 Gewichtsprozent, und höchstens 40, insbesondere 30 Gewichtsprozent verwendet. Wenn die Komponenten c) und/oder d) verwendet werden, beträgt ihr Mindestgehalt vorzugsweise 1, insbesondere 5 Gewichtsprozent, und ihr Höchstgehalt vorzugsweise 20, insbesondere 10 Gewichtsprozent.

In den vorstehenden Formeln (I), (II) und (III) können mehrmals vorhandene Reste R, R′, R″, X bzw. Y bei einer Verbindung jeweils die gleiche oder unterschiedliche Bedeutung haben.

Die Alkylreste bedeuten z.B. geradkettige, verzweigte oder cyclische Reste mit 1 bis 20, vorzugsweise 1 bis 10 Kohlenstoffatomen und insbesondere niedere Alkylreste mit 1 bis 6, vorzugsweise 1 bis 4 Kohlenstoffatomen. Spezielle Beispiele sind Methyl, Ethyl, n-Propyl, Isopropyl, n-Butyl, sek.-Butyl, tert.-Butyl, Pentyl, n-Hexyl und Cyclohexyl. Die Arylreste enthalten z.B. 6 bis 25, vorzugsweise 6 bis 14 und insbesondere 6 bis 10 Kohlenstoffatome. Spezielle Beispiele sind Phenyl und Naphthyl, wobei Phenol bevorzugt ist.

Die Alkenylreste sind z.B. geradkettige, verzweigte oder cyclische Reste mit 2 bis 20, vorzugsweise 2 bis 10 Kohlenstoffatomen und insbesondere niedere Alkenylreste, wie Vinyl, Alkyl und 2-Butenyl.

Die Alkoxy-, Acyloxy-, Alkylamino-, Arylalkyl-, Alkylaryl-, Arylalkenyl-, Alkenylaryl-, Alkylen, Alkylenphenylen-, Keto-, Carbonsäurealkylester- und substituierten Aminoreste leiten sich z.B. von den vorstehend genannten Alkyl-, Alkenyl- und Arylresten ab. Spezielle Beispiele sind Methoxy, Ethoxy, n- und i-Propoxy, n-, sek-, und tert.-Butoxy, Acetyloxy, Propionyloxy, Monomethylamino, Monoethylamino, Dimethylamino, Diethylamino, Monomethylanilino, Benzyl, Tolyl, Methylen, Ethylen, Dimethylen, Toluylen und Styryl.

Die genannten Reste können gegebenenfalls übliche Substituenten tragen, z.B. Halogenatome, niedere Alkylreste, Hydroxy-, Nitro- oder Aminogruppen.

Unter den Halogenen sind Fluor, Chlor und Brom bevorzugt und Chlor besonders bevorzugt.

Bei den organofunktionellen Silanen (b) und den Organosilanen (c) haben a vorzugsweise den Wert 0 und b vorzugsweise den Wert 2.

Spezielle Beispiele für hydrolysierbare Silane (a) sind $SiCl_4$, $HSiCl_3$, $Si(OCH_3)_4$, $Si(OC_2H_5)_4$ und $Si(OOCH_3)_4$.

Bei den organofunktionellen Silanen (b) kann die Brückengruppe R″ gegebenenfalls durch Sauerstoff- oder Schwefelatome oder -NH-Gruppen unterbrochen sein. Vorzugsweise entstehen auf diese Art 2 bis 10 sich wiederholende Struktureinheiten.

Spezielle Beispiele für organofunktionelle Silane (b) sind:

$(C_2H_5O)_3$-Si-$(CH_2)_3$-OH, $(C_2H_5O)_3$-Si-$CH_2$-$NH_2$,
$(CH_3O)_3$-Si-$(CH_2)_3$-NH-$(CH_2)_2$-$NH_2$,
$(C_2H_5O)_3$-Si-p-$C_{64}$-$NH_2$,
$(C_2H_5O)_3$-Si-$(CH_2)_3$-OH, $(CH_3O)_3$-Si-$(CH_2)_4$-SH,
$CH_3(CH_3O)_2$-Si-$CH_2$-$CH(CH_3)$-$CH_2$-NH-$(CH_2)_2$-$NH_2$,
$CH_3(C_2H_5O)_2$-Si-$(CH_2)_4$-$NH_2$, $(CH_3)_2C_2H_5O$-Si-$CH_2$-$NH_2$,
$CH_3(C_2H_5O)_2$-Si-$CH_2$-OH, $(C_3H_7)_3$-Si-$(CH_2)_3$-CN,

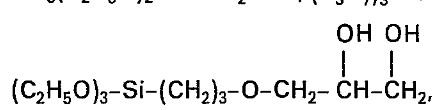,

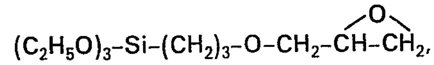,

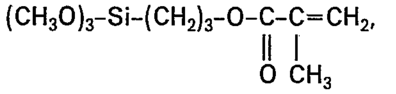

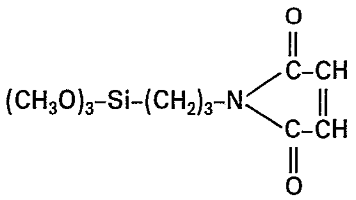

$(CH_3O)_3$-Si-$(CH_2)_3$-S-$(CH_2)_3$-OH

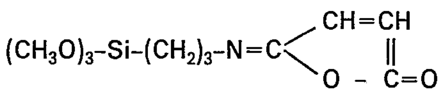

$(CH_3O)_3$-Si-$(CH_2)_3$-S-$(CH_2)_2$-CHO.

Spezielle Beispiele für Organosilane (c) sind:

$(CH_3)_2$-Si-$Cl_2$, $(CH_3)_2$-Si-$(OCH_3)_3$, $(CH_3)_2$-Si-$(OC_2H_5)_2$, $(C_6H_5)_2$-Si-$Cl_2$, $(C_6H_5)_2$-Si-$(OC_2H_5)_2$, $CH_2$=CH-Si-$Cl_3$, $CH_2$=CH-$CH_2$-Si-$(OC_2H_5)_3$, $CH_2$=CH-$CH_2$-Si-$(CH_3COO)_3$, i-$C_3H_7)_3$-Si-OH und $(CH_3)_2$-Si-$(OH)_2$.

Diese Silane sind zum Teil Handelsprodukte oder sie lassen sich nach bekannten Methoden herstellen; vgl. W. Noll, «Chemie und Technologie der Silicone», Verlag Chemie GmbH, Weinheim/Bergstrasse (1968).

Anstelle der monomeren Ausgangssilane (a) bis (c) können gegebenenfalls auch vorkondensierte, im Reaktionsmedium lösliche Oligomere dieser Silane eingesetzt werden; d.h. geradkettige oder cyclische, niedermolekulare Teilkondensate (Polyorganosiloxane) mit einem Kondensationsgrad von z.B. etwa 2 bis 6.

Als Komponente (d) werden im Reaktionsmedium lösliche, schwerflüchtige Oxide oder derartige schwerflüchtige Oxide bildende Verbindungen von Elementen der Hauptgruppen Ia bis Va oder der Nebengruppen IVb oder Vb des Periodensystems eingesetzt. Vorzugsweise leitet sich die Komponente (d) von folgenden Elementen ab: Na, K, Mg, Ca, B, Ti, Zr, Al, Si, Pb, P, As und/oder V, wobei Na, Ca, Mg, B, Al, Ti und P besonders bevorzugt sind.

Unter den schwerflüchtigen Oxiden sind $Na_2O$, $K_2O$, CaO, $TiO_2$, $As_2O_3$ und $P_2O_5$ und $B_2O_3$ besonders bevorzugt.

Im Reaktionsmedium lösliche, schwerflüchtige Oxide bildende Verbindungen sind z.B. anorganische Säuren, wie Phosphorsäure und Borsäure, sowie deren Ester, Halogenide und Salze. Ferner eignen sich z.B. Hydroxide, wie NaOH, KOH oder $Ca(OH)_2$ und Alkoxide, wie NaOR, KOR, $Ca(OR)_2$, Al $(OR)_3$ oder $Ti(OR)_4$, wobei sich R von niederen Alkoholen, wie Methanol, Ethanol, Propanol oder Butanol, ableitet. Weitere verwendbare Ausgangsverbindungen sind entsprechende Salze mit flüchtigen Säuren, z.B. Acetate, basische Acetate, wie basisches Bleiacetat, Formiate, Nitrate und Halogenide.

Die hydrolytische Polykondensation der Ausgangskomponenten erfolgt z.B. in einem organischen Lösungsmittel in Gegenwart einer zur Kondensation ausreichenden Wassermenge, vorzugsweise 50 bis 150% und insbesondere 100% der zur Hydrolyse der vorhandenen hydrolysierbaren Gruppen stöchiometrisch erforderlichen Wassermenge. Beispiele für geeignete organische Lösungsmittel sind Alkohole, vorzugsweise niedere Alkohole, wie Methanol und Ethanol, Ketone, vorzugsweise niedere Dialkylketone, wie Aceton und Methylisobutylketon, Ether, vorzugsweise niedere Dialkylether, wie Diethylether, Amide, wie Dimethylformamid, und deren Gemische.

Gegebenenfalls wird dem Reaktionssystem ein Kondensationskatalysator zugesetzt, z.B. eine Protonen oder Hydroxylionen abspaltende Verbindung oder ein Amin. Beispiele für geeignete Katalysatoren sind organische oder anorganische Säuren, wie Salzsäure, Salpetersäure, Schwefelsäure, Phosphorsäure, Ameisensäure, Essigsäure oder Benzoesäure, sowie organische oder anorganische Basen, wie Ammoniak, Alkali- oder Erdalkalimetallhydroxide, z.B. Natrium-, Kalium- oder Calciumhydroxid, und wasserlösliche Amine, wie Triethylamin. Hierbei sind flüchtige Säuren und Basen, insbesondere Salzsäure und Ammoniak, besonders bevorzugt. 1 N Mineralsäuren sind besonders bevorzugt. Die Katalysatorkonzentration beträgt vorzugsweise $5 \times 10^{-2}$ bis $5 \times 10^{-4}$, insbesondere $10^{-2}$ bis $10^{-3}$ Mol $H^+$ bzw. $OH^-$ pro Liter Reaktionsgemisch.

Die Polykondensation wird üblicherweise bei −20 bis +150°C, vorzugsweise bei Raumtemperatur, bei erhöhtem oder verringertem Druck, vorzugsweise jedoch bei Atmosphärendruck, an der Luft durchgeführt. Gegebenenfalls kann auch unter Inertgasschutz gearbeitet werden.

Nach beendeter Kondensation trennt man das organische Lösungsmittel ab und wäscht das Produkt mit Wasser bei Raumtemperatur oder erhöhter Temperatur frei von Lösungsmittel und Katalysator. Anschliessend kann gegebenenfalls getrocknet werden, z.B. unter vermindertem Druck bei 40 bis 60°C.

Die mechanische Zerkleinerung des Produkts wird in üblichen Mühlen, z.B. Scheibenschwingmühlen oder Kugelmühlen, gegebenenfalls in einem organischen Medium, wie Ethanol, durchgeführt. Aus dem erhaltenen Granulat können dann stabile Suspensionen auf verschiedene Weise hergestellt werden.

Ein einfaches Verfahren ist das Mahlen und Sieben auf die gewünschte Korngrösse und anschliessende Suspendieren in einem wässrigen Medium. Ein bevorzugtes Verfahren besteht jedoch darin, dass man das Granulat in Wasser aufschlämmt, gegebenenfalls Peptisierungsmittel, wie Triethanolamin, Natriumcarbonat oder Ammoniumoxalat, zugibt und die Aufschlämmung z.B. 5 Minuten bis 24 Stunden mechanisch rührt. Anschliessend lässt man z.B. 1 bis 10 Minuten absetzen und dekantiert die Suspension von der sedimentierten Grobfraktion ab. Die erhaltene Suspension kann dann zu einem Pulver gefriergetrocknet oder als solches weiter verarbeitet werden.

Ein alternatives Verfahren besteht darin, das zerkleinerte Granulat in organischen Medien von unterschiedlicher Dichte zu sedimentieren, um auf diese Weise die gewünschte Fraktionierung zu erzielen. Hierbei werden z.B. für die Feinstteilchen Ether, für die Mittelfraktion Ethanol und für die Grobfraktion Chloroform oder Kohlenstofftetrachlorid oder entsprechende Lösungsmittelgemische verwendet. Nach dem Abtrennen der gewünschten Korngrössenfraktion wird das Lösungsmittel z.B. in einem Rotationsverdampfer abgedampft und man erhält das gewünschte pulverförmige Produkt.

Die Ankopplung der biochemischen Materialien, z.B. Antigene, Antikörper, Hormone, Aminosäuren, Haptene, Proteine oder Enzyme, an die Kieselsäureheteropolykondensat-Teilchen erfolgt nach üblichen Methoden der organischen und Biochemie. Geeignete Verfahren sind in der einschlägigen Fachliteratur und z.B. der US-PS 3 652 761 beschrieben.

Je nach der Reaktivität der funktionellen Gruppen der anzukoppelnden biochemischen Materialien bzw. des Kieselsäureheteropolykondensats müssen die funktionellen Gruppen des Kieselsäureheteropolykondensats gegebenenfalls nach den Methoden der Organischen Chemie weiter modifiziert werden. Hierbei kann es erforderlich sein, zunächst das Kieselsäureheteropolykondensat zu derivatisieren und anschliessend das gewünschte Material anzukoppeln. Als Derivatisierungsmittel kommen z.B. Amine, Carbonsäuren, Säurechloride, Thiocarbamate, Thiocarbaminsäurechlorid, Diazoverbindungen, Ester oder Sulfide in Betracht.

Die Wahl der Modifizierung hängt dabei von den notwendigen Bedingungen der nachfolgenden Kopplungsreaktion ab, da hierbei in Temperatur-, pH-Bereichen und Medien gearbeitet werden muss, bei denen eine irreversible Denaturierung oder ein Aktivitätsverlust der biochemischen Materialien weitgehend ausgeschlossen ist bzw. bei denen die charakteristischen Eigenschaften dieser Materialien nicht wesentlich verändert werden. Vorzugsweise werden die biochemischen Materialien deshalb bei Raumtemperatur in einem wässrigen Medium bei pH 2 bis 11 angekoppelt. Andererseits ist darauf zu achten, dass die Kopplungsreaktion mit genügender Geschwindigkeit abläuft, um die biochemischen Materialien nicht zu lange nicht-physiologischen Bedingungen auszusetzen.

Unter Beachtung dieser Gesichtspunkte lassen sich Kieselsäureheteropolykondensate herstellen bzw. modifizieren, die nach Ankopplung des biochemischen Materials optimale Resultate liefern. Eine Modifizierung eines γ-Aminopropylgruppen enthaltenden Kondensats kann z.B. dadurch erfolgen, dass man das Kondensat 30 bis 60 Minuten mit einer wässrigen, etwa 2,5prozentigen Glutaraldehydlösung behandelt. Das Diazoderivat des genannten Kondensats kann z.B. durch Umsetzen mit p-Nitrobenzoylchlorid, Reduzieren der Nitrogruppe zur Aminogruppe und Diazotieren mit Salpetriger Säure hergestellt werden. Wenn das Kieselsäureheteropolykondensat aufgrund der Verwendung geeigneter funktioneller Silane als Ausgangsverbindungen bereits Anilinogruppen enthält, kann sofort mit Salpetriger Säure diazotiert werden. Durch Umsetzen von Aminogruppen des Kondensats mit Thiophosgen gelangt man zum Isothiocyanoderivat.

Andererseits ist bei Verwendung von z.B. Aldehydgruppen oder Maleinimidogruppen enthaltenden Ausgangssilanen keine weitere Modifizierung des Kieselsäureheteropolykondensats erforderlich und die biochemischen Materialien können direkt angekoppelt werden. Bei Carboxylgruppen aufweisenden Kondensaten empfiehlt es sich, die Kopplungsreaktion in Gegenwart von Carbodiimid durchzuführen.

Die gegebenenfalls derivatisierten Kieselsäureheteropolykondensate werden zur Ankopplung in einem geeigneten Puffersystem, z.B. Citrat (pH 2 bis 3), Acetat (pH 4,0), Phosphat (pH 7,0), Bicarbonat (pH 9,5) oder Borat (pH 8,0) mit dem biochemischen Material inkubiert. Hierbei werden zwischen dem Kieselsäureheteropolykondensat und den biochemischen Materialien kovalente Bindungen geknüpft. Die Inkubationszeit beträgt im allgemeinen 10 Minuten bis 24 Stunden bei Raumtemperatur. Zum Entfernen des Überschusses von biochemischem Material werden die Kieselsäureheteropolykondensat-Teilchen dann abfiltriert oder abzentrifugiert und mit Wasser oder Pufferlösung gewaschen und können dann in einem gewünschten wässrigen Medium zu den erfindungsgemässen Suspensionspräparaten suspendiert werden.

Die Suspensionspräparate werden in geeignete Behälter abgefüllt und können an der Luft oder unter Inertgas gelagert werden. Sie können für die verschiedensten Anwendungszwecke eingesetzt werden, z.B. als Trennmittel in immunchemischen Verfahren, wie Radio-, Enzym- oder Fluoreszenzimmunoassays, zur Affinitätschromatographie oder in anderen biologischen Verfahren, z.B. als Bioreaktoren in der Enzymtechnik oder Mikrobiologie.

Für die Fixierung und Stabilisierung mercaptogruppenhaltiger biologischer und biochemischer Materialien haben sich neuartige Imino- und Imidosilane der allgemeinen Formel IV

$$Y'(CH_2)_cSiX'_3 \qquad (IV)$$

in der Y' eine N-Maleinimido- oder N-(5-Iminofuran-2-on)-Gruppe ist, X' Alkoxy mit 1 bis 4, vorzugsweise 1 bis 2 Kohlenstoffatomen bedeutet und c eine ganze Zahl von 2 bis 10, vorzugsweise 3 bis 6 ist, sowie deren Homopolykondensate und Copolykondensate mit einer oder mehreren der vorstehend genannten anderen Komponenen a) bis d) als besonders wirksam erwiesen. Gegenstand der Erfindung sind deshalb auch diese

neue Silane (IV) und ihre Homo- und Copolykondensate.

Zur Fixierung und Stabilisierung Mercaptogruppen enthaltender biochemischer Materialien können die monomeren Silane (IV) oder ihre Homo- oder Copolykondensate auf übliche Träger aufgebracht werden, z.B. auf Glas, Keramikstoffe, Kunststoffe, Metalle, Metalloxide oder Papiere, die vorzugsweise vorher gereinigt wurden. Alternativ kann man die Homo- und Copolykondensate auch als erfindungsgemässe Suspensionspräparate einsetzen, nachdem auf die vorstehend beschriebene Weise die biochemischen Materialien kovalent angekoppelt wurden.

Die folgenden Beispiele erläutern die Erfindung, ohne sie zu beschränken.

Beispiel 1

Herstellung von Aldehydgranulat

160 g Tetramethoxysilan und 40 g 3-Oxopropyl-3'-trimethoxysilylpropylthioether, 300 ml Methanol und 42 g Wasser sowie 9,3 g 1 N HCl werden in dieser Reihenfolge in einem offenen Becherglas vorgelegt und 17 Stunden kondensiert. Man erhält ein hartes, glasartiges Granulat, das mit 70°C heissem Wasser bis zur pH-Konstanz (pH 6) ausgewaschen wird. Anschliessend wird im Trokkenschrank bei 50°C vorgetrocknet und im Ölpumpenvakuum nachgetrocknet.

Zum Zerkleinern werden 20 g Granulat 20 Minuten in einer Scheibenschwingmühle und anschliessend 3 Stunden in einer Kugelmühle mit Ethanol gemahlen. Das Lösungsmittel wird in einem Rotationsverdampfer abgezogen, wobei ein pulverförmiges Produkt zurückbleibt.

Beispiel 2

Herstellung von Maleinimidogranulat

Nach dem Verfahren von Beispiel 1 werden 1,01 Mol Tetramethoxysilan, 0,076 Mol 3-Hydroxypropyl-3'-trimethoxysilylpropylthioether, 0,074 Mol γ-Maleinimidopropyltriethoxysilan, 372 ml Methanol und 40,1 ml Wasser in ein Becherglas gegeben und 17 Stunden stehengelassen. Man erhält ein festes körniges Produkt, das gemäss Beispiel 1 weiter verarbeitet wird.

Beispiel 3

Herstellung eines Anilinogranulats

Nach dem Verfahren von Beispiel 1 werden 0,53 Mol Tetramethoxysilan und 0,06 Mol N-p-Aminobenzoyl-3-aminopropyltriethoxysilan, 150 ml Methanol und 21 ml Wasser sowie 4,65 ml 1 N HCl zusammengegeben und über Nacht stehengelassen. Das erhaltene körnige Produkt wird neutral gewaschen und gemäss Beispiel 1 zerkleinert.

Beispiel 4

Herstellung eines Aminogranulats

Nach dem Verfahren von Beispiel 1 werden 0,53 Mol Tetramethoxysilan und je 0,04 Mol γ-Aminopropyltriethoxysilan bzw. 3-Hydroxypropyl-3'-trimethoxysilylpropylthioether, 150 ml Methanol und 21 ml Wasser in einem offenen Becherglas zusammengegeben und 17 Stunden kondensiert. Das anfallende Kondensat wird neutral gewaschen und gemäss Beispiel 1 weiter verarbeitet.

Beispiel 5

Herstellung stabiler Suspensionen

1 g der zerkleinerten Granulate wird in 100 ml Wasser aufgeschlämmt, in einen Erlenmayer-Kolben gegeben und mit einem Quirl 10 Minuten gerührt. Anschliessend werden die Bestandteile, die sich innerhalb von 5 Minuten absetzen, abgetrennt und die verbleibende Suspension gefriergetrocknet. Das auf diese Weise erhaltene Pulver kann in wässrigen Lösungen zu stabilen Suspensionen dispergiert werden.

Beispiel 6

Kopplung eines T4-Antikörpers an Anilinogranulat

Zur Diazotierung werden 100 ml 4N HCl und 1 g Natriumnitrit mit 4 g Anilinogranulat 10 Minuten bei 4°C gerührt und dann solange mit jeweils 100 ml 0,1 m Bicarbonatpuffer von pH 8,4 gespült, bis der pH-Wert 8,4 beträgt. Das gewaschene Granulat wird in 30 ml-Fläschchen gefüllt und mit 20 ml 0,1 m Bicarbonatpuffer (pH 8,4) und 150 µl nativem T4-Antiserum versetzt. Hierauf rollt man 90 Minuten auf dem Rollblock, zentrifugiert, giesst den Überstand ab und wäscht mit 0,01 m Phosphatpuffer von pH 7,4, bis der pH-Wert 7,4 erreicht ist. Dann wird wieder zentrifugiert und der Überstand abgegossen, worauf man das gewaschene Granulat in 60 ml-Fläschchen einfüllt und mit 0,01 m Phosphatpuffer von pH 7,4 aufschlämmt. Zur Ermittlung der Standardkurven werden jeweils 5, 10, 25, 50 bzw. 100 µl mit T4-Antikörper gekoppeltes Granulat zu 1 ml T4-Tracer gegeben und die Standardkurven gemessen.

Beispiel 7

T3-Assay

1 g Aldehydgranulat mit einer Korngrösse von 0,050 bis 0,063 mm wird mit 20 ml T3-Antiserum (Verdünnung 1:150 000) in 0,1 Mol/l Citratpuffer (pH 3) 30 Minuten unter Rotation bei Raumtemperatur inkubiert. Nach Entfernen der Kopplungslösung wird zweimal mit destilliertem Wasser gewaschen. Anschliessend wird das Antikörper-Granulat (AK-Granulat) in 2 ml 0,05 Mol/l Trispuffer (pH 8,9) aufgeschlämmt und in einem T3 RIA eingesetzt.

Dazu werden 100 µl T3-Standard, 500 µl $^{125}$I-T3-Lösung und 100 µl AK-Granulat in ein Teströhrchen pipettiert, mit einem Vibrationsmischer vermischt und 2,5 h bei 37°C/Wasserbad inkubiert. Anschliessend wird 15 Minuten bei 2000 × g zentrifugiert, der Überstand mit einer Pasteurpipette entfernt und der Rückstand im Gammazähler gemessen. Es wird eine Bindekapazität von 19% erhalten; die Diskriminierung zwischen ersten und letztem Standardpunkt beträgt 56,5%.

Beispiel 8

FT4-Assay

1,5 g Aldehydgranulat mit einer Korngrösse von 0,050 bis 0,063 mm werden mit 25 ml 10% BSA in 0,1 Mol/l Citratpuffer (pH 3) unter Rotation 30 Minuten bei Raumtemperatur inkubiert. Nach Entfernen der BSA-Lösung wird zweimal in destilliertem Wasser gewaschen und in 6 ml 0,01 Mol/l Phosphatpuffer pH 7,4 aufgeschlämmt.

Für den Assay werden verwendet:

FT4-Standards enthaltend 60, 125, 250, 500, 1000, 2000 pg/ml T4 in 0,01 Mol/l Phosphatpuffer (pH 7,4) $^{125}$I-T4-Lösung (ca. 120 000 IpM/ml) T4-Antiserum (RIA-mat T4) 2.AK/PEG-Lösung (Verdünnung 1:500)

Assay-Fliessschema

200 µl Standard/Patientenserum
200 µl Granulat (50 mg) mischen, 2 h bei 37°C/Wasserbad inkubieren

↓

15 Minuten zentrifugieren

↓

Überstand entfernen

↓

mit 500µl Phosphatpuffer (37°C) waschen

↓

15 Minuten zentrifugieren

↓

Überstand entfernen

↓

500 µl $^{125}$I-T4-Lösung + 50 µl T4-Antiserum

↓

mischen, 25 Minuten bei RT inkubieren

↓

500 µl 2.AK/PEG-Lösung

↓

mischen, 15 Minuten bei RT inkubieren

↓

30 Minuten zentrifugieren

↓

Überstand entfernen

↓

Rückstand messen

Es wird eine Bindungskapazität von 50,6% erhalten, die Diskriminierung zwischen erstem und letztem Standardpunkt beträgt ca. 11 000 IpM.

Beispiel 9

Herstellung von γ-Maleinimidopropyltrimethoxysilan

In einen Dreihalskolben mit Rückflusskühler und zwei Tropftrichtern werden unter Schutzgas und magnetischem Rühren 675 ml Methanol und 26,2 g (0,27 Mol) Maleinsäureimid gegeben. In den Tropftrichtern werden 50 ml Methanol und 49,6 ml (0,27 Mol) 30prozentige Natriummethylatlösung bzw. 400 ml Methanol und 63,2 g (0,26 Mol) γ-Brompropyltrimethoxysilan vorgelegt. Die Maleinimidlösung wird zum Rückfluss erhitzt. In der Siedehitze wird die Methylatlösung während 1 h zugetropft. Dann tropft man unter Rückfluss während 3 h die Silanlösung zu. Nach 17stündigem Stehen bei Raumtemperatur wird das Lösungsmittel im Ölpumpenvakuum am Rotationsverdampfer abgezogen und der zähflüssige, rote Rückstand in 1 l Ether aufgenommen, wobei ein feinkristalliner Niederschlag entsteht. Man filtriert mit einer Umkehrfritte unter Schutzgas. Nach Abziehen des Lösungsmittels erhält man eine rotgefärbte Flüssigkeit, aus der die gewünschte Verbindung isoliert wird (Ausbeute 20%).

IR (Film): 2844 ($OCH_3$), 1715 (C = O), 1085 (Si-O), 820 (Si-OC) $cm^{-1}$.

GC-MS: 150°C, 4 Peaks,

Peak 1: γ-Brompropyltrimethoxysilan

Peak 2: γ-Maleinimidopropyltrimethoxysilan

MS: m/z = 259 ($M^+$; 20%)

ber. ($C_{10}H_{17}NO_5Si$) 259,08771, gef. 259,08911,

Peak 3: höhermolekulares, bromhaltiges, nicht identifiziertes Produkt,

Peak 4: höhermolekulares, nicht identifiziertes Produkt.

Beispiel 10

Herstellung von 5-(3′-Triethoxysilylpropyl)-iminofuran-2-on

In einen 1 l Dreihalskolben mit Rückflusskühler werden unter Schutzgas und magnetischem Rühren nacheinander 500 ml Ether, 41 g (0,2 Mol) N,N′-Dicyclohexylcarbodiimid, 20 g (0,2 Mol) Maleinsäureanhydrid und 47 g (0,2 Mol) γ-Aminopropyltriethoxysilan gegeben. Es wird weitere 17 h bei Raumtemperatur gerührt. Der ausgefallene N,N′-Dicyclohexylharnstoff wird in einer Umkehrfritte unter Schutzgas abfiltriert und die klare, gelbgefärbte Lösung am Rotationsverdampfer am Ölpumpenvakuum abgezogen. Das erhaltene Produkt wird fraktioniert destilliert. Man erhält 50 g eines konstant siedenden Produktes.

Sdp.: 111–113°C bis 0,12–0,2 mbar.

IR (Film): 3100 (= C-H), 1800 (C = O), 1692 (C = N), 1581 (C = C), 1080 (Si-O), 790 (Si-OC) $cm^{-1}$.

MS: m/z = 301 ($M^+$, 2%)

ber. ($C_{13}H_{23}NO_5Si$) 301,13469, gef. 301,13325.

90MHZ-$^1$H-NMR ($CDCl_3$): $\delta$ = 7,34 ($J_{AB}$ = 5,5 Hz), 3,83 (q, $^3J$ = 7,2 Hz, 6H), 3,71-3,49 (m, 2H), 2,02-1,55 (m, 2H), 1,21 (t, $^3J$ = 7,2 Hz, 9H), 0,82-0,57 ppm (m, 2H).

## Patentansprüche

1. Stabilisierte biochemische Suspensionspräparate, die Teilchen eines funktionelle Gruppen aufweisenden Trägers, an dessen funktionelle Gruppen biochemische Materialien kovalent gebunden sind, in einem wässrigen Medium suspendiert enthalten, dadurch gekennzeichnet, dass sie als Träger Kieselsäureheteropolykondensat-Teilchen enthalten, die hergestellt worden sind durch hydrolytische Polykondensation von

a) mindestens einem vollständig hydrolysierbaren Silan der allgemeinen Formel I

$$SiX_4 \qquad (I)$$

in der X Wasserstoff, Halogen, Hydroxy, Alkoxy, Acyloxy oder $-NR_2$ (R = Wasserstoff und/oder Alkyl) bedeutet, jedoch nicht alle Reste X Wasserstoff sind, und

b) mindestens einem organofunktionellen Silan der allgemeinen Formel II

$$R'_a(R''Y)_bSiX_{(4-a-b)} \quad (II)$$

in der X die vorstehende Bedeutung hat, R' Alkyl, Alkenyl, Aryl, Arylalkyl, Alkylaryl, Arylalkenyl oder Alkenylaryl bedeutet, R'' Alkylen, Phenylen, Alkylenphenylen oder Alkenylen darstellt, wobei diese Reste durch Sauerstoff- oder Schwefelatome oder -NH-Gruppen unterbrochen sein können, Y Halogen oder eine gegebenenfalls substituierte Amino-, gegebenenfalls substituierte Anilino-, Amid-, Polyamid-, Aldehyd-, Keto-, Carboxy-, Hydroxy-, Polyol-, Mercapto-, Cyano-, Diazo-, Carbonsäure-alkylester-, Sulfonsäure-, Phosphorsäure-, Acryloxy-, Methacryloxy-, Epoxid-, Vinyl-, N-Maleinimido- oder N-(5-Iminofuran-2-on)-Gruppe ist, a den Wert 0, 1 oder 2 und b den Wert 1, 2 oder 3 haben, wobei (a + b) den Wert 1, 2 oder 3 hat sowie gegebenenfalls den folgenden Komponenten c) und/oder d):

c) mindestens einem Organosilan der allgemeinen Formel III

$$R'_bSiX_{(4-b)} \quad (III)$$

in der X, R' und b die vorstehende Bedeutung haben;

d) mindestens einem im Reaktionsmedium löslichen schwer-flüchtigen Oxid oder mindestens einer im Reaktionsmedium löslichen, ein schwerflüchtiges Oxid bildenden Verbindung eines Elements der Hauptgruppen Ia bis Va oder der Nebengruppen IVb oder Vb des Periodensystems, in einem organischen Lösungsmittel gebenenfalls in Gegenwart eines Kondensationskatalysators, wobei das Kieselsäureheteropolykondensat 40 bis 95 Gewichtsprozent der Komponente a), 5 bis 50 Gewichtsprozent der Komponente b), 0 bis 40 Gewichtsprozent der Komponente c) und 0 bis 30 Gewichtsprozent der Komponente d), jeweils bezogen auf Siloxan- bzw. Oxideinheiten des Polykondensats, enthält.

2. Präparate nach Anspruch 1, dadurch gekennzeichnet, dass sie 0,1 bis 50 Gewichtsprozent Kieselsäureheteropolykondensat-Teilchen, bezogen auf das Gewicht der Suspension, enthalten.

3. Präparate nach Anspruch 1 oder 2, dadurch gekennzeichnet, dass das Kieselsäureheteropolykondensat eine Teilchengrösse von 0,1 bis 10 µm hat.

4. Präparate nach einem der Ansprüche 1 bis 3, dadurch gekennzeichnet, dass sie zusätzlich Suspensionshilfsmittel, Puffer, Konservierungsmittel und/oder andere übliche Additive enthalten.

5. Verwendung der Präparate nach einem der Ansprüche 1 bis 4 in immunchemischen Verfahren wie z.B. Radio-, Enzym- oder Fluoreszenzimmunoassays oder der Affinitätschromatographie.

**Claims**

1. Stabilized suspended biochemical preparations which contain, suspended in an aqueous medium, particles of a carrier having functional groups to which biochemical materials are covalently bound, characterized in that they contain, as a carrier, particles of a silicic acid heteropolycondensate produced by hydrolytic polycondensation of

a) at least one completely hydrolyzable silane of the general formula I

$$SiX_4 \quad (I)$$

wherein X is hydrogen, halogen, hydroxy, alkoxy, acyloxy or $-NR_2$ (R = hydrogen and/or alkyl), with the proviso that not all of radicals X are hydrogen, and

b) at least one organo-functional silane of the general formula II

$$R'_a(R''Y)_bSiX_{(4-a-b)} \quad (II)$$

wherein X is as defined above, R' is alkyl, alkenyl, aryl, arylalkyl, alkylaryl, arylalkenyl or alkenylaryl, R'' is alkylene, phenylene, alkylenephenylene or alkenylene, said radicals being optionally interrupted by oxygen or sulphur atoms or -NH-groups, Y is halogen or an optionally substituted amino, optionally substituted anilino, amide, polyamide, aldehyde, keto, carboxy, hydroxy, polyol, mercapto, cyano, diazo, carboxylic acid alkyl ester, sulphonic acid, phosphoric acid, acryloxy, methacryloxy, epoxy, vinyl, N-maleinimido or N-(5-iminofuran-2-one) group, a is 0, 1 or 2 and b is 1, 2 or 3, (a + b) having a value of 1, 2 or 3, as well as, optionally, the following components c) and/or d):

c) at least one organo-silane of the general formula III

$$R'_bSiX_{(4-b)} \quad (III)$$

wherein X, R' and b are as defined above;

d) at least one substantially involatile oxide of an element of the main groups Ia to Va or the secondary groups IVb or Vb or the periodic table, which is soluble in the reaction medium, or at least one compound which is soluble in the reaction medium and is capable of forming such a substantially involatile oxide, in an organic solvent, optionally, in the presence of a condensation catalyst, said silicic acid heteropolycondensate containing 40 to 95 percent by weight of component a), 5 to 50 percent by weight of component b), 0 to 40 percent by weight of component c) and 0 to 30 percent by weight of component d), each based on the siloxane or oxide units of the polycondensate.

2. Preparation according to claim 1, which contain 0,1 to 50 percent by weight, based on the weight of the suspension, of silicic acid heteropolycondensate particles.

3. Preparations according to claim 1 or 2, wherein the silicic acid heteropolycondensate has a particle size of from 0,1 to 10 μm.

4. Preparations according to any one of claims 1 to 3 which additionally contain suspension adjuvants, buffers, preservatives and/or other conventional additives.

5. The use of the preparations according to any one of claim 1 to 4 in immunochemical methods such as, for example, radio, enzyme or fluorescence immunoassays, or in affinity chromatography.

**Revendications**

1. Préparations biochimiques stabilisés en suspension, qui contiennent des particules d'un support présentant des groupes fonctionnels et aux groupes tonctionnels duquel des matières biochimiques sont fixées par covalence, préparations caractérisées en ce qu'elles contiennent comme supports des particules d'un hétéropolycondensat d'acide silicique, qui ont été préparées par polycondensation hydrolytique:

a) d'au moins un silane entièrement hydrolysable, de formule générale I:

$$SiX_4 \quad (I)$$

dans laquelle X représente un atome d'hydrogène ou d'halogène ou un groupe hydroxy, alcoxy, acyloxy ou $-NR_2$ (R étant un atome d'hydrogène et/ou un groupe alkyle), mais tous les restes R ne représentant pas de l'hydrogène, et

b) d'au moins un silane organofonctionnel de formule générale II:

$$R'_a(R''Y)_bSiX_{(4-a-b)} \quad (II)$$

dans laquelle X a le sens précité, R' représente un groupe alkyle, alcényle, aryle, arylalkyle, alkylaryle, arylalcényle ou alcénylaryle, R" représente un groupe alkylène, phénylène, alkylènephénylène ou alcénylène, les restes pouvant être interrompus par des atomes d'oxygène ou de soufre ou par des groupes -NH-, Y représente un atome d'halogène ou un groupe amino éventuellement substitué, anilino éventuellement substitué, amide, polyamide, aldéhyde, céto, carboxy, hydroxy, polyol, mercapto, cyano, diazo, ester alkylique d'acide carboxylique, acide sulfonique, acide phosphorique, acryloxy, méthacryloxy, époxyde, vinyle, N-maléinimido ou N-(5-imino-furanne-2-one), a vaut 0, 1 ou 2, et b vaut 1, 2 ou 3, la somme (a + b) valant 1, 2 ou 3, ainsi que, éventuellement, des composants c) et/ou d) suivants:

c) au moins un organosilane répondant à la formule générale III:

$$R'_bSiX_{(4-b)} \quad (III)$$

dans laquelle X, R' et b ont le sens précité;

d) au moins un oxyde peu volatil, soluble dans le milieu de réaction ou au moins un composé, formant un oxyde peu volatil et soluble dans le milieu de réaction, d'un élément des groupes principaux Ia à Va ou des sous-groupes IVb ou Vb du Système Périodique, dans un solvant organique, éventuellement en présence d'un catalyseur de condensation, l'hétéropolycondensat de l'acide silicique contenant 40 à 95% en poids du composant a), 5 à 50% en poids du composant b), 0 à 40% en poids du composant c) et 0 à 30% en poids du composant d), les proportions étant chaque fois rapportées aux motifs siloxane ou oxyde du polycondensat.

2. Préparations selon la revendication 1, caractérisées en ce qu'elles contiennent 0,1 à 50% en poids de particules d'un hétéropolycondensat d'acide silicique, sur la base du poids de la suspension.

3. Préparations selon la revendication 1 ou 2, caractérisées en ce que l'hétéropolycondensat d'acide silicique présente une grandeur de particules de 0,1 à 10 μm.

4. Préparations selon l'une des revendications 1 à 3, caractérisées en ce qu'elles contiennent en outre un adjuvant de suspension, un tampon, un agent de conservation et/ou d'autres additifs usuels.

5. Utilisation des préparations selon l'une des revendications 1 à 4 dans un procédé immunochimique, comme par exemple des dosages radioimmunologiques, immuno-enzymatiques ou d'immunofluorescence, ou pour la chromatographie d'affinité.